# FASCICULE DE BREVET EUROPEEN

(11) **EP 2 836 119 B1**
(45) Date de publication et mention de la délivrance du brevet: **03.03.2021**
(21) Numéro de dépôt: 13720473.1
(22) Date de dépôt: 11.04.2013
(51) Int. Cl.: A61B 5/117

(54) **DISPOSITIF MÉDICAL POUR LA MESURE ET LE TRAITEMENT D'UN PARAMÈTRE DE SANTÉ D'UN PATIENT**
MEDIZINISCHE VORRICHTUNG ZUR MESSUNG UND VERARBEITUNG EINES GESUNDHEITSPARAMETERS EINES PATIENTEN
MEDICAL DEVICE FOR THE MEASUREMENT AND PROCESSING OF A HEALTH PARAMETER OF A PATIENT

(30) Priorité: 12.04.2012 FR 1253359
(43) Date de publication de la demande: 18.02.2015
(73) Titulaire: MARQUE VERTE SANTE, 54500 Vandoeuvre Les Nancy (FR)
(72) Inventeur: CHAPUSOT, Thierry, F-54850 Messein (FR)
(74) Mandataire: Rhein, Alain
(86) Numéro de dépôt international: PCT/FR2013/050784
(87) Numéro de publication internationale: WO 2013/153334

(56) Documents cités:
- US-A1- 2003 173 408
- US-A1- 2006 075 257
- US-A1- 2007 017 136
- US-A1- 2007 213 603
- US-A1- 2008 097 551
- US-A1- 2009 043 180
- US-A1- 2010 182 126
- US-A1- 2010 182 126
- US-A1- 2011 092 825
- US-A1- 2011 093 298
- US-B1- 6 975 961

## Description

### Domaine technique et état de l'art

L'invention concerne un dispositif médical pour la mesure à distance et le traitement d'un paramètre de santé d'un patient. L'invention permet le suivi médical à distance d'un patient.

On trouve déjà dans le commerce des dispositifs médicaux portables et faciles d'utilisation pour la mesure de paramètre de santé d'un patient. Ces dispositifs comprennent notamment un dispositif de mesure d'un paramètre, par exemple une température, une tension artérielle, etc., et un dispositif d'affichage du résultat de la mesure. Ces dispositifs permettent à un patient de mesurer lui-même certains paramètres de santé et de suivre l'évolution de ses paramètres de santé ; ils permettent également au patient d'informer son médecin traitant des résultats des mesures effectuées. Certains de ces dispositifs comprennent également des moyens pour transmettre le résultat de la mesure à un serveur distant auquel le médecin peut avoir accès.

Une difficulté de ces dispositifs est la sécurisation des données du patient et, pour le médecin, la nécessité de s'assurer de l'identité du patient sur lequel les mesures ont été effectuées.

Certains des dispositifs médicaux existants ont un accès limité : pour activer le dispositif, un utilisateur doit fournir un identifiant, un mot de passe, etc. Mais une fois le dispositif activé, toute personne peut utiliser le dispositif de sorte que rien ne garantit que la personne sur laquelle sont effectuées les mesures de paramètre est bien celle associée au mot de passe. De plus, de tels dispositifs sont nécessairement réservés à l'usage d'une seule personne puisqu'un unique identifiant, un unique mot de passe, etc. permet l'activation du dispositif.

L'invention vise à pallier tout ou partie des inconvénients des dispositifs médicaux existants.

### Description de l'invention

L'invention concerne un dispositif médical comprenant un dispositif de mesure d'un paramètre de santé d'un patient, un dispositif de prise d'une empreinte identifiant le patient, un dispositif de communication avec un serveur distant et un moyen de commande. De manière connue, dans un tel dispositif, le moyen de commande est adapté pour, lorsqu'il est activé par une unique action du patient :
- activer le dispositif de mesure d'un paramètre de santé du patient, et obtenir en retour un paramètre mesuré,
- activer le dispositif de prise d'empreinte et obtenir en retour une empreinte prise identifiant le patient.
- fournir le paramètre mesuré et l'empreinte prise au dispositif de communication pour transmission du paramètre mesuré et de l'empreinte prise au serveur distant.

Conformément à l'invention, le dispositif de prise d'empreinte est un dispositif de prise d'une empreinte digitale, comprenant un écran tactile agencé pour activer le moyen de commande lorsqu'une présence d'un doigt est détectée sur l'écran tactile, et un moyen de copie agencé pour prendre une image de l'empreinte d'un doigt en contact avec l'écran tactile lorsque le moyen de copie est activé par le moyen de commande.

L'avantage d'une telle caractéristique est que l'activation du moyen de commande et la prise d'empreinte se font simultanément ou quasi-simultanément : lors d'une unique application du doigt du patient sur l'écran tactile, l'écran tactile active le moyen de commande, le moyen de commande active le moyen de copie et obtient en retour une image de l'empreinte du doigt en appui sur l'écran tactile. L'utilisation du dispositif médical selon l'invention est ainsi très simple pour le patient car l'utilisation du dispositif médical, jusqu'à et y compris l'envoi des résultats au serveur distant est entièrement automatique, le patient intervenant uniquement pour activer le moyen de commande. Autre avantage d'un dispositif de prise d'une empreinte digitale : un tel dispositif est aujourd'hui largement répandu, fiable, efficace et peu cher.

Le dispositif médical selon l'invention permet ainsi la mesure d'un paramètre de santé du patient, la prise d'une empreinte du patient sur lequel la mesure est effectuée, puis surtout l'envoi ensemble sur un serveur distant du paramètre mesuré et de l'empreinte prise, le tout sur une unique action du patient. Ceci permet de garantir l'identité de la personne sur laquelle la mesure a été effectuée. De plus, dans la mesure où une empreinte est prise systématiquement à chaque nouvelle utilisation du dispositif médical, un même dispositif médical peut être utilisé par des personnes différentes, et il n'est pas nécessaire de mémoriser dans le dispositif médical l'empreinte du patient, ni même un identifiant ou un mot de passe. Ainsi le dispositif médical selon l'invention ne peut être utilisé pour accéder de manière frauduleuse à des informations confidentielles d'un patient. De plus, l'utilisation du dispositif médical selon l'invention est très simple pour le patient car l'utilisation du dispositif médical, jusqu'à et y compris l'envoi des résultats au serveur distant, est entièrement automatique, le patient intervenant uniquement pour activer le moyen de commande.

La prise d'empreinte du patient peut être réalisée avant, simultanément ou après la mesure du paramètre de santé, l'important étant que :
- le dispositif de mesure et le dispositif de prise d'empreinte sont activés par une unique action du patient pour garantir que le paramètre mesuré et l'empreinte prise correspondent à un même patient,
- le paramètre mesuré et l'empreinte prise sont disponibles ensemble au moment de la transmission au serveur distant pour un traitement simultané ultérieur.

De préférence, le dispositif de mesure et le dispositif de prise d'empreinte sont activés simultanément par le dispositif de commande, ou quasi simultanément, par exemple avec moins de cinq secondes (et de préférence moins de une seconde) entre l'activation du dispositif de mesure et l'activation du dispositif de prise d'empreinte. Dans ces conditions, il n'est matériellement pas possible que l'empreinte prise par le dispositif de prise d'empreinte appartienne à une personne autre que la personne sur laquelle le dispositif de mesure agit.

A réception d'une transmission, le serveur distant peut notamment :
- à partir de l'empreinte prise, identifier un patient référencé dans une base de données de patients, ou émettre un signal d'alerte si le patient n'est pas référencé,
- à partir du paramètre mesuré, valider que le dit paramètre est dans une plage de valeur attendue pour le patient identifié ou émettre un signal d'alerte sinon.

Les signaux de validation ou les signaux d'alerte peuvent ensuite être transmis à une personne identifiée (un médecin, une infirmière, un proche, etc.) pour un traitement approprié (visite du patient, proposition de rendez-vous au patient, etc.).

Selon des variantes, dans le dispositif médical selon l'invention, le dispositif de mesure d'un paramètre de santé est par exemple, mais non limitativement :
- un dispositif de mesure du poids du patient, et / ou
- un dispositif de mesure d'une tension du patient, et / ou
- un dispositif de mesure d'un taux de créatinine dans le sang du patient, et / ou
- un dispositif de mesure d'un taux de glycémie dans le sang du patient, et / ou
- un dispositif de mesure d'un taux de prothrombine dans le sang du patient, et / ou
- un dispositif de mesure d'une température du patient, et / ou
- un dispositif de mesure d'un rythme cardiaque du patient.

Bien sûr, cette liste de dispositifs de mesure n'est pas exhaustive. Plus généralement, le dispositif de mesure du dispositif médical selon l'invention peut être tout dispositif de mesure connu à ce jour, ou susceptible d'être développé dans le futur, apte à mesurer un paramètre de santé d'un patient.

Bien sûr, un dispositif médical selon l'invention peut comprendre plusieurs dispositifs de mesure ; dans ce cas, le moyen de commande est de préférence adapté pour, lorsqu'il est activé par le patient :
- activer les dispositifs de mesure d'un paramètre de santé du patient, successivement ou simultanément, et obtenir en retour un paramètre mesuré d'au moins certains des dispositifs de mesure,
- activer le dispositif de prise d'empreinte et obtenir en retour une empreinte prise, puis,
- fournir les paramètres mesurés et l'empreinte prise au dispositif de communication pour transmission au serveur distant.

Le dispositif de prise d'une empreinte permet d'identifier de manière unique une personne.

Selon un mode de réalisation, le dispositif de communication peut comprendre un dispositif de mémorisation d'un paramètre de connexion à un réseau de communication tel qu'un réseau de téléphonie ou un réseau de type Internet, et un dispositif de transmission, et le moyen de commande est également adapté pour :
- lire dans le dispositif de mémorisation un paramètre de connexion et fournir le paramètre de connexion et une demande de connexion au dispositif de transmission qui transmet au réseau de communication,
- associer le paramètre mesuré et l'empreinte prise en un message unique, et fournir le message unique au dispositif de transmission qui transmet au serveur via le réseau de communication.

Le moyen de commande peut également être adapté pour, en complément des deux étapes précédentes :
- lire dans le dispositif de mémorisation un identifiant d'abonnement à un service de santé,
- associer le paramètre mesuré, l'empreinte prise et l'identifiant d'abonnement en un message unique, et fournir le message unique au dispositif de transmission qui transmet au serveur via le réseau de communication.

Le dispositif de mémorisation des paramètres de connexion et de l"identifiant d'abonnement est par exemple une carte SIM ou une zone prédéfinie dans un dispositif de stockage de données du dispositif médical. Le dispositif de transmission peut être relié au réseau de communication avec ou sans fil. Dans le cas d'un réseau sans fil, le dispositif de transmission peut comprendre, selon le cas et de manière connue en soi, une antenne d'émission / réception de données, et un modem : le modem est adapté pour convertir une donnée, un message ou une instruction reçue du moyen de commande en un format adapté au réseau de communication et transmettre la donnée, le message ou l'instruction convertie sur l'antenne.

Le moyen de commande peut également être adapté pour chiffrer le message avant transmission au serveur distant. Ceci interdit toute exploitation malveillante du message unique.

Le moyen de communication peut également comprendre un dispositif de réception d'un message de validation de réception émis par le serveur distant. Le dispositif de communication peut comprendre en complément un dispositif de stockage du message de validation, ou un dispositif d'affichage du dit message de validation, comme par exemple un écran d'affichage, un témoin lumineux ou un moyen d'affichage d'un signal sonore (haut-parleur, ...) ; le moyen de commande est selon le cas adapté pour mémoriser le message de validation dans le dispositif de stockage, ou pour afficher, sur le dispositif d'affichage, le message de validation reçu du serveur distant. Le patient est ainsi informé que ses données ont bien été reçues par le serveur distant.

Le moyen de commande peut également être adapté pour réactiver automatiquement le dispositif de communication en cas d'échec d'une transmission du paramètre mesuré et de l'empreinte prise au serveur distant, plusieurs fois si nécessaire, jusqu'à réception du message de validation de réception émis par le serveur distant.

Le dispositif médical selon l'invention peut encore comprendre un dispositif de stockage, tel qu'une mémoire réinscriptible et le moyen de commande est adapté pour mémoriser dans le dispositif de stockage le paramètre mesuré et l'empreinte prise, ou le message unique, selon des variantes. De préférence, le moyen de commande est également adapté pour effacer du dispositif de stockage le paramètre mesuré et l'empreinte prise, ou le message unique, après transmission au serveur distant. Ainsi après transmission, les informations confidentielles ne peuvent être exploitées.

Les paramètres de connexion correspondent à un abonnement au réseau de téléphonie utilisé pour le transfert de données ; ils sont fournis par le gestionnaire du réseau de communication. L'identifiant d'abonnement est un identifiant délivré au patient (ou au professionnel de santé proposant le dispositif médical au patient) lors d'un abonnement à un service de santé gérant l'utilisation de dispositifs médicaux selon l'invention ; l'identifiant d'abonnement est par exemple enregistré par un professionnel de santé dans le dispositif de mémorisation (par ex. carte SIM) du dispositif médical selon l'invention lors de la fourniture du dispositif médical au patient ; en parallèle, le professionnel de santé qui attribue le dispositif médical au patient transmet au gestionnaire de service médical diverses informations telles que : un identifiant du dispositif médical délivré au patient, un identifiant du patient ou des paramètres identifiant le patient de manière unique (nom, adresse, etc.), une empreinte (de type correspondant à celles susceptibles d'être prises par le dispositif médical) du patient, un identifiant du professionnel de santé attribuant le dispositif médical au patient, un identifiant des professionnels de santé habilités à consulter les informations relatives au patient sur les bases de données du gestionnaire du service de santé, etc.

### Brève description des figures

L'invention sera mieux comprise, et d'autres caractéristiques et avantages de l'invention apparaîtront à la lumière de la description qui suit d'un exemple de réalisation d'un dispositif médical selon l'invention, exemple donné à titre non limitatif. La description est à lire en relation avec les dessins annexés dans lesquels :
- la figure 1 montre un schéma d'utilisation d'un dispositif médical selon l'invention,
- la figure 2 est une vue extérieure d'une partie d'un dispositif selon l'invention, et
- la figure 3 détaille le boîtier de commande d'un dispositif selon l'invention.

### Description d'un mode de réalisation de l'invention

Comme dit précédemment, un dispositif médical selon l'invention comprend un ou plusieurs dispositifs de mesure d'un paramètre de santé d'un patient, un dispositif de prise d'une empreinte 20 identifiant le patient, un dispositif de communication 30 avec un serveur distant 40 et un moyen de commande 50.

Le dispositif de prise d'empreinte 20, le dispositif de communication 30 et le moyen de commande 50 sont dans l'exemple représenté regroupés ensemble dans un même boîtier 100 de commande, qui peut être de petite taille, par exemple la taille d'un téléphone mobile ou d'une tablette numérique.

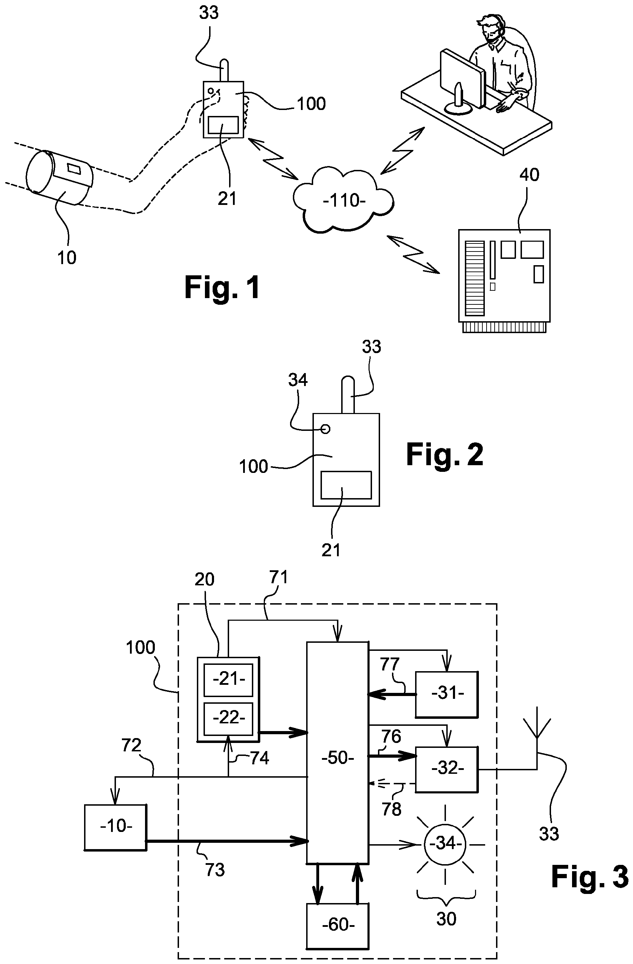

Sur la figure 3 sont détaillés les principaux éléments intégrés dans le boîtier de commande. Les flèches en trait fin indiquent le sens de transmission d'instruction entre deux éléments, et les flèches en trait épais indiquent le sens de transmission de données entre deux éléments.

Dans l'exemple représenté, le dispositif de mesure est un tensiomètre comprenant notamment un brassard 10 destiné à être fixé au bras d'un patient et, dans le boîtier de commande 50, le moyen de commande 50 est relié au brassard 10. Dans un autre exemple, non représenté, le dispositif médical est par exemple un fauteuil, sur lequel le patient peut s'installer, fauteuil intégrant plusieurs moyens de mesure, par exemple un mesureur de poids, un boîtier de prise de sang et d'analyse de certains paramètres du sang comme par exemple un taux de créatine, un mesureur de température positionné au niveau d'une zone d'appui de la tête du patient sur le dossier du fauteuil, etc. Le boîtier de commande de l'ensemble est par exemple positionné sur un bras du fauteuil, pour être accessible manuellement par le patient.

Selon les dimensions et la forme du (ou des) moyen(s) de mesure, le (ou les) moyen(s) de mesure peut (peuvent) être dans le même boîtier de commande 100, ou bien peut (peuvent) être extérieur(s) au boîtier, relié(s) au moyen de commande par une liaison, filaire ou non, permettant l'échange de données numériques (instructions d'activation et résultats de mesure, etc.).

Dans l'exemple représenté, le moyen de prise d'une empreinte 20 est un moyen de prise d'une empreinte digitale comprenant un écran tactile 21 et un moyen de copie agencé pour prendre une image de l'écran tactile 21. Lorsque le patient positionne un doigt sur l'écran tactile 21, l'écran tactile active le moyen de commande 50 qui active immédiatement le moyen de copie de l'empreinte du doigt présent sur l'écran tactile 21.

Le ou les moyens de mesure fournissent le paramètre mesuré sous la forme d'un signal numérique, exploitable directement par le moyen de communication. De la même façon, le moyen de prise d'empreinte fournit une empreinte sous la forme d'un signal numérique exploitable par le moyen de communication.

Lorsqu'il est activé par le patient (flèche 71), le moyen de commande 50 du dispositif médical selon l'invention est agencé pour :
- activer le dispositif de mesure 10 (flèche 72), et obtenir en retour un paramètre mesuré (flèche 73), dans l'exemple une valeur de la tension artérielle du patient
- activer le dispositif de prise d'empreinte 20 (flèche 74) et obtenir en retour une empreinte prise (flèche 75), puis,
- fournir le paramètre mesuré et l'empreinte prise au dispositif de communication 30 (76) pour transmission du paramètre mesuré et de l'empreinte prise au serveur distant 40.

Dans l'exemple représenté, le dispositif de communication 30 comprend quant à lui un dispositif de mémorisation 31, un dispositif de transmission, un dispositif de réception et un dispositif d'affichage 34, pilotés par le moyen de commande 50 de la manière suivante :
- le moyen de commande lit dans le dispositif de mémorisation 31 un paramètre de connexion (flèche 77) et fournit le paramètre de connexion et une demande de connexion au dispositif de transmission qui transmet au réseau de communication 110,
- le moyen de commande associe le paramètre mesuré et l'empreinte prise en un message unique, chiffre le message unique et fournit le message chiffré au dispositif de transmission qui transmet au serveur 40 via le réseau de communication 110,
- le dispositif de réception reçoit du serveur distant 40 un message de validation de réception (flèche 78), et
- le moyen de commande 50 transmet le message de validation reçu au dispositif d'affichage 34 qui l'affiche.

Dans l'exemple représenté, le dispositif de mémorisation 31 est par exemple une carte SIM, qui contient des paramètres d'identification d'un abonné à un réseau de communication tel qu'un réseau de téléphonie ou un réseau de type Internet. Le dispositif de transmission et le dispositif de réception comprennent ensemble une antenne 32 et un modem 33. Le dispositif d'affichage 34 est un témoin lumineux, qui affiche par exemple une couleur rouge après activation du dispositif médical et jusqu'à réception du message de validation, puis affiche une couleur verte après réception du message de validation.

Le paramètre mesuré et l'empreinte prise peuvent être associés par un simple aboutement (mise bout à bout) des deux signaux numériques, par une modulation de l'un avec l'autre, ou par toute autre association mathématique souhaitée et connue dans le domaine du traitement des signaux numériques.

Dans l'exemple représenté encore le dispositif médical comprend un dispositif de stockage 60, tel qu'une mémoire inscriptible. Le dispositif de commande 50 est adapté pour mémoriser dans le dispositif de stockage 60 le message unique, puis pour effacer du dispositif de stockage le message unique, après transmission du paramètre mesuré et de l'empreinte prise, ou du message unique au serveur distant.

### NOMENCLATURE

- 10: dispositif de mesure
- 20: dispositif de prise d'empreinte
21 écran tactile
- 30: dispositif de communication
31 dispositif de mémorisation
32 modem
33 antenne
34 témoin lumineux
- 40: serveur distant
- 50: moyen de commande
- 60: dispositif de stockage
- 100: boîtier de commande
- 110: réseau de communication

## Revendications

1. Dispositif médical comprenant un dispositif de mesure d'un paramètre de santé d'un patient, un dispositif de prise d'une empreinte identifiant le patient, un dispositif de communication avec un serveur distant et un moyen de commande, le moyen de commande étant adapté pour, lorsqu'il est activé par une unique action du patient :
• activer le dispositif de mesure d'un paramètre de santé du patient, et obtenir en retour un paramètre mesuré,
• activer le dispositif de prise d'empreinte et obtenir en retour une empreinte prise, puis,
fournir le paramètre mesuré et l'empreinte prise au dispositif de communication pour transmission du paramètre mesuré et de l'empreinte prise au serveur distant, **caractérisé en ce que** le dispositif de prise d'empreinte est un dispositif de prise d'une empreinte digitale, comprenant un écran tactile agencé pour activer le moyen de commande lorsqu'une présence d'un doigt est détectée sur l'écran tactile, et un moyen de copie agencé pour prendre une image de l'empreinte d'un doigt en contact avec l'écran tactile lorsque le moyen de copie est activé par le moyen de commande..

2. Dispositif selon la revendication 1, dans lequel le dispositif de commande active simultanément le dispositif de mesure et le dispositif de prise d'empreinte.

3. Dispositif médical selon l'une des revendications précédentes, dans lequel le dispositif de mesure d'un paramètre de santé est :
• un dispositif de mesure du poids du patient, et / ou
• un dispositif de mesure d'une tension du patient, et / ou
• un dispositif de mesure d'un taux de créatinine dans le sang du patient, et / ou
• un dispositif de mesure d'un taux de glycémie dans le sang du patient, et / ou
• un dispositif de mesure d'un taux de prothrombine dans le sang du patient, et / ou
• un dispositif de mesure d'une température du patient, et / ou
• un dispositif de mesure d'un rythme cardiaque du patient.

4. Dispositif médical selon l'une des revendications précédentes, dans lequel le dispositif de communication comprend un dispositif de mémorisation d'un paramètre de connexion à un réseau de communication tel qu'un réseau de téléphonie ou un réseau de type Internet, et un dispositif de transmission, et dans lequel le moyen de commande est également agencé pour :
• lire dans le dispositif de mémorisation un paramètre de connexion et fournir le paramètre de connexion et une demande de connexion au dispositif de transmission qui transmet au réseau de communication,
• associer le paramètre mesuré et l'empreinte prise en un message unique, et fournir le message unique au dispositif de transmission qui transmet au serveur via le réseau de communication.

5. Dispositif selon la revendication 4, dans lequel le moyen de commande est également agencé pour :
• lire dans le dispositif de mémorisation un identifiant d'abonnement à un service de santé,
• associer le paramètre mesuré, l'empreinte prise et l'identifiant d'abonnement en un message unique, et fournir le message unique au dispositif de transmission qui transmet au serveur via le réseau de communication.

6. Dispositif médical selon la revendication 5, dans lequel le moyen de commande est également agencé pour chiffrer le message unique avant transmission au serveur distant.

7. Dispositif selon l'une des revendications 5 ou 6, dans lequel le dispositif de communication comprend également un dispositif de réception d'un message de validation de réception émis par le serveur distant.

8. Dispositif selon la revendication 7, comprenant également un dispositif d'affichage, tel qu'un écran d'affichage, un témoin lumineux ou un moyen d'affichage d'un message sonore, et dans lequel le moyen de commande est adapté pour afficher, sur le dispositif d'affichage, le message de validation de réception reçu du serveur distant.

9. Dispositif selon l'une des revendications précédentes, comprenant également un dispositif de stockage, tel qu'une mémoire, et dans lequel le dispositif de commande est adapté pour mémoriser dans le dispositif de stockage le paramètre mesuré et l'empreinte prise, ou le message unique.

10. Dispositif selon la revendication précédente, dans lequel le moyen de commande est également agencé pour effacer du dispositif de stockage le paramètre mesuré et l'empreinte du patient, ou le message unique, après transmission du paramètre mesuré et de l'empreinte prise, ou du message unique au serveur distant.

## Patentansprüche

1. Medizinische Vorrichtung, umfassend eine Vorrichtung zum Messen eines Gesundheitsparameters eines Patienten, eine Vorrichtung zum Aufnehmen eines Abdrucks, der den Patienten identifiziert, eine Vorrichtung zum Kommunizieren mit einem Fernserver und ein Steuermittel, wobei das Steuermittel angepasst ist, wenn es durch eine einzelne Aktion des Patienten aktiviert ist, um:
• die Vorrichtung zum Messen eines Gesundheitsparameters des Patienten zu aktivieren und darauffolgend einen gemessenen Parameter zu erhalten,
• die Vorrichtung zum Aufnehmen eines Abdrucks zu aktivieren und darauffolgend einen aufgenommenen Abdruck zu erhalten, danach
den gemessenen Parameter und den aufgenommenen Fingerabdruck an die Kommunikationsvorrichtung zu liefern, für die Übertragung des gemessenen Parameters und des aufgenommenen Abdrucks an den Fernserver, **dadurch gekennzeichnet, dass** die Vorrichtung zum Aufnehmen eines Abdrucks eine Vorrichtung zum Aufnehmen eines Fingerabdrucks ist, die einen Touchscreen umfasst, der angeordnet ist, um das Steuermittel zu aktivieren, wenn ein Vorhandensein eines Fingers auf dem Touchscreen erfasst wird, und eine Kopiervorrichtung, die angeordnet ist, um ein Bild des Abdrucks eines mit dem Touchscreen in Berührung stehenden Fingers aufzunehmen, wenn das Kopiermittel durch das Steuermittel aktiviert ist.

2. Vorrichtung nach Anspruch 1, bei der die Steuervorrichtung die Messvorrichtung und die Vorrichtung zum Aufnehmen eines Abdrucks gleichzeitig aktiviert.

3. Medizinische Vorrichtung nach einem der vorhergehenden Ansprüche, bei der die Vorrichtung zum Messen eines Gesundheitsparameters:
• eine Vorrichtung zum Messen des Gewichts eines Patienten, und/oder
• eine Vorrichtung zum Messen eines Blutdrucks des Patienten, und/oder
• eine Vorrichtung zum Messen des Kreatininspiegels im Blut des Patienten, und/oder
• eie Vorrichtung zum Messen des Blutzuckerspiegels im Blut des Patienten, und/oder
• eine Vorrichtung zum Messen eines Prothrombinspiegels im Blut des Patienten, und/oder
• eine Vorrichtung zum Messen einer Temperatur des Patienten, und/oder
• eine Vorrichtung zum Messen einer Herzfrequenz des Patienten, ist.

4. Medizinische Vorrichtung nach einem der vorhergehenden Ansprüche, bei der die Kommunikationsvorrichtung eine Vorrichtung zum Speichern eines Parameters der Verbindung mit einem Kommunikationsnetzwerk, wie einem Telefonnetz oder einem Internet-Netzwerk, und eine Übertragungsvorrichtung umfasst, und bei der das Steuermittel auch angeordnet, um:
• einen Verbindungsparameter vom Speichergerät zu lesen und den Verbindungsparameter und eine Verbindungsanforderung an die Übertragungsvorrichtung zu liefern, die an das Kommunikationsnetzwerk weiterleitet,
• den gemessenen Parameter und den aufgenommenen Abdruck in einer einzelnen Nachricht zu verknüpfen und die einzelne Nachricht an die Übertragungsvorrichtung zu liefern, die über das Kommunikationsnetzwerk an den Server weiterleitet.

5. Vorrichtung nach Anspruch 4, bei der das Steuermittel auch angeordnet ist, um:
• eine Identifizierung eines Abonnements für einen Gesundheitsservice von der Speichervorrichtung zu lesen,
• den gemessenen Parameter, den aufgenommenen Abdruck und die Identifizierung des Abonnements in einer einzelnen Nachricht zu verknüpfen und die einzelne Nachricht an die Übertragungsvorrichtung zu liefern, die über das Kommunikationsnetzwerk an den Server weiterleitet.

6. Medizinische Vorrichtung nach Anspruch 5, bei der das Steuermittel auch angeordnet ist, um die einzelne Nachricht vor der Übertragung an den Fernserver zu verschlüsseln.

7. Vorrichtung nach einem der Ansprüche 5 oder 6, bei der die Kommunikationsvorrichtung auch eine Vorrichtung zum Empfangen einer von dem Fernserver gesendeten Empfangsbestätigungsnachricht umfasst.

8. Vorrichtung nach Anspruch 7, umfassend auch eine Anzeigevorrichtung, wie einen Anzeigebildschirm, eine Anzeigelampe oder ein Mittel zum Anzeigen einer akustischen Nachricht, bei der das Steuermittel angepasst ist, um auf der Anzeigevorrichtung die vom Fernserver empfangene Empfangsbestätigungsnachricht anzuzeigen.

9. Vorrichtung nach einem der vorhergehenden Ansprüche, umfassend auch eine Speichervorrichtung, wie einen Speicher, bei der die Steuervorrichtung angepasst ist, um den gemessenen Parameter und den aufgenommenen Abdruck, oder die einzelne Nachricht in die Speichervorrichtung zu speichern.

10. Vorrichtung nach dem vorhergehenden Anspruch, bei der das Steuermittel auch angeordnet ist, um den gemessenen Parameter und den Abdruck des Patienten, oder die einzelne Nachricht nach Übertragung des gemessenen Parameters und des aufgenommenen Abdrucks, oder der einzelnen Nachricht an den Fernserver aus der Speichervorrichtung zu löschen.

## Claims

1. Medical device comprising a device for measuring a health parameter of a patient, a device for taking an imprint identifying the patient, a device for communicating with a remote server and a control means, the control means being suitable for, when it is activated by a single action by the patient:
• activating the device for measuring a health parameter of a patient, and obtaining, in return, a measured parameter,
• activating the device for taking an imprint and obtaining, in return, an impression being taken, then
providing the measured parameter and the imprint being taken to the communication device for transmission of the measured parameter and the imprint being taken to the remote server, wherein the device for taking an imprint is a device for taking a fingerprint, comprising a touch screen arranged to activate the control means when a presence of a finger is detected on the touch screen, and a means for copying arranged to take an image of the imprint of a finger into contact with the touch screen when the means for copying is activated by the control means.

2. Device according to claim 1, wherein the control device activates the measuring device and the device for taking an imprint simultaneously.

3. Medical device according to one of the preceding claims, wherein the device for measuring a health parameter is:
• a device for measuring the weight of the patient, and/or
• a device for measuring the blood pressure of the patient, and/or
• a device for measuring a creatinine level in the blood of the patient, and/or
• a device for measuring a blood glucose level in the blood of the patient, and/or
• a device for measuring a prothrombin level in the blood of the patient, and/or
• a device for measuring a temperature of the patient, and/or
• a device for measuring a heart rate of the patient.

4. Medical device according to one of the preceding claims, wherein the communication device comprises a device for storing a parameter for connecting to a communication network such as a telephone network or a network such as Internet, and a transmission device, and in which the control means is also arranged for:
• reading from the storage device a connection parameter and providing the connection parameter and a connection request to the transmission device, which transmits to the communication network,
• associating the measured parameter and the imprint being taken in a single message, and providing the single message to the transmission device, which transmits to the server through the communication network.

5. Device according to claim 4, wherein the control means is also arranged for:
• reading from the storage device an identifier of subscription to a health service,
• associating the measured parameter, the imprint being taken and the subscription identifier in a single message, and providing the single message to the transmission device, which transmits to the server through the communication network.

6. Medical device according to claim 5, wherein the control means is also arranged to encrypt the single message before transmission to the remote server.

7. Device according to one of claims 5 or 6, wherein the communication device also comprises a device for receiving a reception validation message sent by the remote server.

8. Device according to claim 7, also comprising a display device, such as a display screen, an indicator light or a means for displaying a sound message, and in which the control means is adapted to display, on the display device, the reception validation message received from the remote server.

9. Device according to one of the preceding claims, also comprising a storage device, such as a memory, and in which the control device is adapted to store in the storage device the measured parameter and the imprint being taken, or the single message.

10. Device according to the preceding claim, wherein the control means is also arranged to erase from the storage device the measured parameter and the imprint of the patient, or the single message, after transmission of the measured parameter and the imprint being taken, or the single message to the remote server.
